# FASCICULE DE BREVET EUROPEEN

(11) **EP 1 139 919 B1**
(45) Date de publication et mention de la délivrance du brevet: **01.09.2004**
(21) Numéro de dépôt: 99964731.6
(22) Date de dépôt: 29.12.1999
(51) Int. Cl.: A61F 2/14, A61F 2/16

(54) **DISPOSITIF POUR TRAITER LA PRESBYTIE OU AUTRE AFFECTION OCULAIRE**
VORRICHTUNG ZUR BEHANDLUNG DER WEITSICHTIGKEIT ODER ANDERER AUGENBESCHWERDEN
DEVICE FOR TREATING PRESBYOPIA OR OTHER OCULAR DISORDER

(30) Priorité: 31.12.1998 FR 9816723
(43) Date de publication de la demande: 10.10.2001
(73) Titulaire: Societe Medicale de Precision S.M.P. SA, 1228 Plan-les-Ouates (CH)
(72) Inventeur: GANEM, Stéphane, F-75007 Paris (FR); STUBLER, Jérôme, F-92200 Neuilly sur Seine (FR); BOS, Gilles, F-74330 La Balme de Sillingy (FR)
(74) Mandataire: Dronne, Guy
(86) Numéro de dépôt international: PCT/FR1999/003307
(87) Numéro de publication internationale: WO 2000/040174

(56) Documents cités:
- EP-A- 0 544 948
- EP-A- 0 732 090
- WO-A-94/02084
- WO-A-95/03755
- WO-A-99/17684
- WO-A-99/17691
- DE-U- 29 801 281

## Description

La présente invention a pour objet un dispositif destiné à être mis en place dans l'oeil pour traiter la presbytie ou d'autres affections oculaires liées à un défaut d'accommodation de l'oeil.

Le document EP0732090A décrit un implant intraoculaire selon le préambule de la revendication 1.

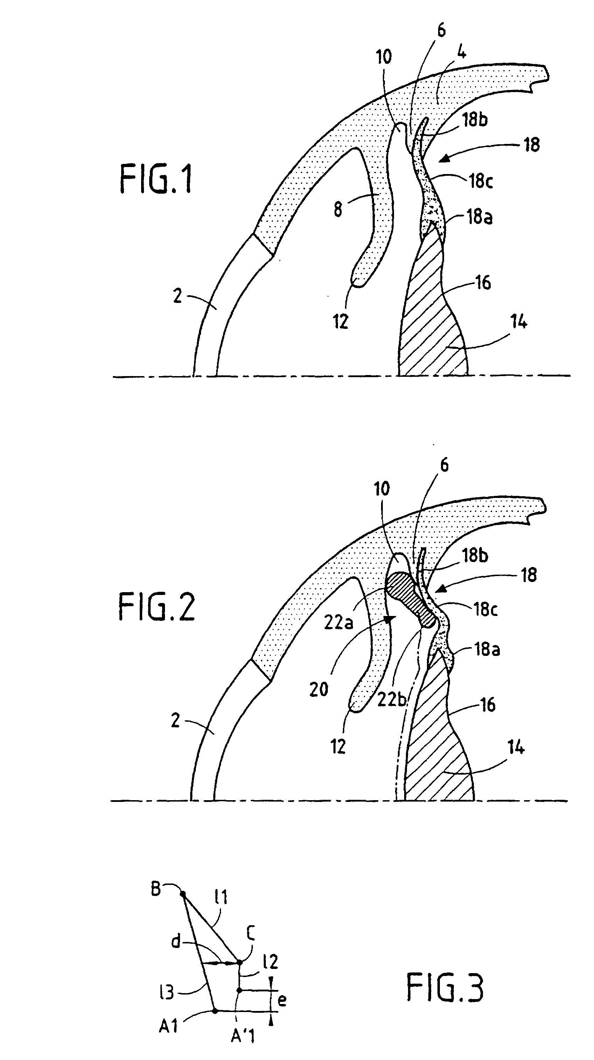

Afin de permettre de mieux comprendre le problème à résoudre, on a représenté sur la figure 1 annexée une demi-vue en coupe verticale d'un oeil. Sur cette figure, on a représenté la cornée 2, la paroi interne de l'oeil 4 avec son corps ciliaire 6 et l'iris 8 qui définit la pupille 9 de l'oeil. Sur cette figure, on a également représenté le sulcus qui constitue un sillon entre le corps ciliaire 6 et l'iris 8 ainsi que le cristallin 14 avec son sac capsulaire 16. Le cristallin 14 ou, plus précisément, son sac capsulaire 16 est relié à la paroi 4 de l'oeil par un ensemble de muscles appelés zonules 18 constitués par des fibrilles. Ces fibrilles ont une extrémité 18a qui est reliée à la périphérie du sac capsulaire 16 et une autre extrémité qui est noyée dans le corps ciliaire 6. Lorsque l'oeil est dans un état normal, les contractions commandées des zonules 18 provoquent la modification des rayons de courbure du cristallin 14 permettant ainsi l'accommodation de l'oeil en fonction de la distance à laquelle se trouve l'objet à regarder.

Il a été mis en évidence que le vieillissement de l'oeil tendait à produire une augmentation du diamètre extérieur du cristallin. Il en résulte que les zonules deviennent "trop longues" et sont "détendues" et que les impulsions appliquées aux fibrilles des zonules ne permettent plus à celles-ci d'agir sur le cristallin pour provoquer l'accommodation.

Il a également été mis en évidence que c'est la traction exercée sur le sac capsulaire par les zonules qui permet d'augmenter la puissance optique du cristallin en provoquant une diminution du rayon de courbure de sa face postérieure.

Un objet de la présente invention est de fournir un dispositif implantable dans l'oeil qui permet de rendre à nouveau les zonules actives pour permettre l'accommodation malgré l'augmentation du diamètre du cristallin.

Pour atteindre ce but, selon l'invention, le dispositif pour traiter la presbytie ou autre affection oculaire liée à un défaut d'accommodation de l'oeil se caractérisé en ce qu'il comprend une pièce ayant sensiblement la forme d'au moins une portion d'anneau présentant un axe de révolution et un premier bord disposé sur un cercle de diamètre D1 compris entre 12,5 et 13,5 mm et un deuxième bord disposé sur un cercle de diamètre D2 compris entre 9,5 et 10,5 mm, lesdits bords étant décalés selon la direction dudit axe de révolution d'une longueur h comprise entre 0,5 et 2,5 mm, ledit premier bord étant destiné à être en appui, par au moins une partie de sa longueur, sur une partie de la paroi interne de l'oeil et ledit deuxième bord étant apte à être appliqué contre une zone médiane des zonules du cristallin, par quoi on obtient un déplacement de ladite zone médiane desdites zonules provoquant leur mise sous tension et les rendant aptes à provoquer la déformation du cristallin sous l'effet de stimulations appliquées aux dites zonules.

On comprend que l'anneau ou la portion d'anneau mis en place à l'intérieur de l'oeil prend appui par son bord externe dans la zone du sulcus ciliaire alors que son autre bord intérieur appliqué contre la zone médiane des zonules provoque le déplacement vers l'arrière de cette zone médiane des zonules ainsi que du cristallin. Ce déplacement permet d'obtenir une nouvelle tension des zonules qui seront ainsi rendues à nouveau actives lorsque les fibrilles qui les constituent seront excitées.

Selon un premier mode de réalisation de l'invention, l'anneau est fermé et il est alors réalisé en un matériau souple biocompatible afin de permettre l'insertion de l'anneau à l'intérieur de l'oeil. Selon un deuxième mode de réalisation de l'invention, l'anneau est ouvert et il peut, dans ce cas, être réalisé en un matériau biocompatible rigide tel que par exemple le PMMA.

Dans le présent texte, il faut préciser que par "matériau souple", on entend des matériaux couramment utilisés pour fabriquer notamment les implants intraoculaires et qui sont typiquement constitués par des polyHEMA ou par des gels de silicone. Par le terme "matériau rigide", il faut entendre des matériaux translucides biocompatibles présentant un faible coefficient d'élasticité et dont le représentant le plus connu est le PMMA.

D'autres caractéristiques et avantages de l'invention apparaîtront mieux à la lecture de la description qui suit de modes de réalisation de l'invention donnés à titre d'exemples non limitatifs. La description se réfère aux figures annexées, sur lesquelles :
- la figure 1 déjà décrite montre la moitié d'un oeil en coupe verticale ;
- la figure 2 montre la mise en place dans l'oeil du dispositif de traitement de la presbytie ;
- la figure 3 est un schéma illustrant le mode d'action du dispositif de traitement de la presbytie ;
- la figure 4 est une vue de face d'un mode de réalisation du dispositif de traitement de la presbytie ;
- la figure 5 est une vue de côté en coupe selon la ligne V-V du dispositif de la figure 4 ; et
- la figure 5a est une vue en coupe partielle de la ligne A-A de la figure 4.

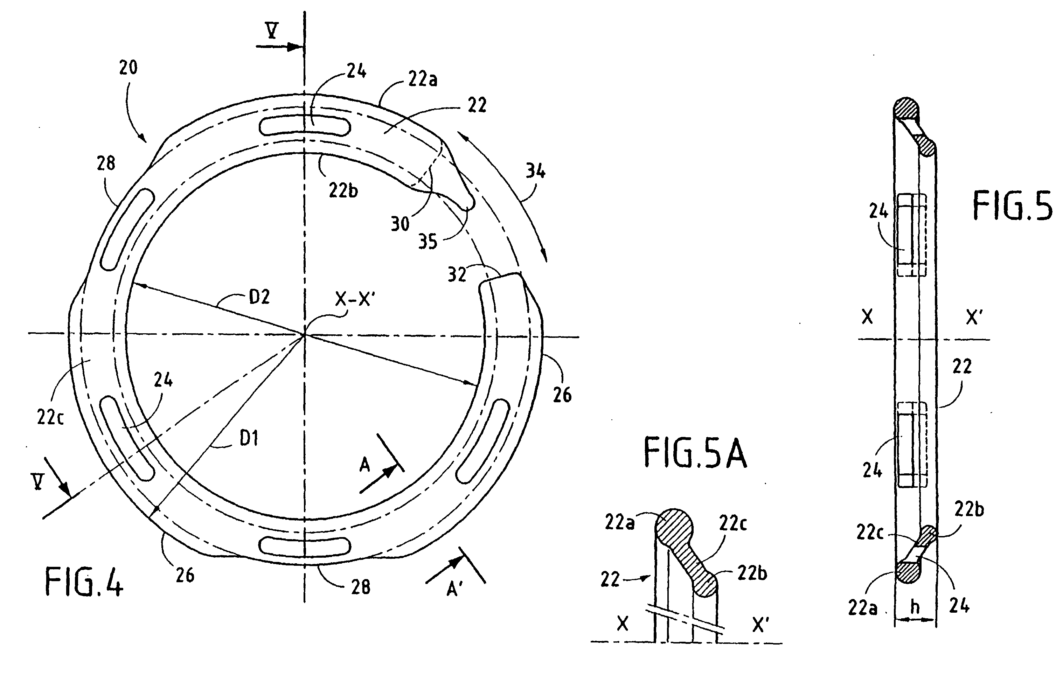

En se référant tout d'abord aux figures 4 et 5, on va décrire un mode de réalisation du dispositif de traitement de la presbytie. Ce dispositif référencé 20 a la forme générale d'un anneau ou d'une portion d'anneau 22 comportant un bord externe 22a et un bord interne 22b. L'anneau 22 peut être fermé ou présenter une ouverture comme cela apparaît sur la figure 4. Les bords 22a et 22b peuvent présenter une forme arrondie et sont reliés entre eux par une portion plane 22c.

Sur la figure 2, on a représenté un dispositif de traitement 20 mis en place dans l'oeil. Comme le montre cette figure, le bord externe 22a est en appui sur la paroi de l'oeil dans la zone du sulcus ciliaire 10 alors que le bord interne 22b est appliqué contre la zone médiane 18 des zonules 18c de manière à provoquer le déplacement de cette zone médiane comme on l'expliquera ultérieurement.

La zone intermédiaire 22c de l'anneau ou de la portion d'anneau 22 doit avoir des dimensions suffisantes pour que le bord interne 22b permette effectivement le déplacement de la zone médiane des zonules. C'est-à-dire que la partie intermédiaire 22c doit présenter une résistance mécanique suffisante pour que la distance entre les bords externe et interne reste constante lorsque l'anneau est mis en place. Ces dimensions dépendront bien sûr du matériau utilisé pour obtenir la résistance mécanique souhaitée. Comme le montre la figure 2 ou la figure 5a. l'anneau 22 a la forme générale d'une portion de tronc de cône de telle manière que le bord externe 22a soit disposé sur un cercle de diamètre D1 que le bord interne 22b soit disposé sur un cercle de diamètre D2 inférieur à D1 et qu'un décalage h selon la direction de l'axe optique X-X' existe entre le bord externe 22a et le bord interne 22b.

Lorsque le dispositif de traitement est mis en place dans l'oeil, le bord 22a constitue un bord antérieur et le bord 22b un bord postérieur.

Sur la figure 3, on a représenté schématiquement l'effet produit par la mise en place du dispositif de traitement 20 dans l'oeil. Sur cette figure, on a symbolisé par B un point d'ancrage fictif des zonules dans le corps ciliaire et par A1 le point d'ancrage des zonules sur la périphérie du sac capsulaire, 13 représentant la distance entre les points B et A1 en l'absence du dispositif de traitement. Par l'action du bord interne 22b sur la zone médiane des zonules, les fibrilles constituant les zonules voient leur partie médiane C déplacée d'une distance d vers l'arrière de l'oeil. Ce déplacement de la zone médiane C provoque également un déplacement du point A1 qui est alors appelé A'₁. Le déplacement selon la direction de l'axe optique est égal à d et on obtient également un déplacement selon des directions orthogonales à l'axe optique, c'est-à-dire selon les directions radiales du cristallin, de valeur e. On comprend que ces déplacements antéro-postérieurs provoquent un certain déplacement vers l'arrière de l'ensemble du cristallin et permettent une mise sous tension des zonules grâce au déplacement de la zone médiane. Tout se passe, par rapport au cristallin, comme si la longueur des zonules avait été réduite de la longueur e. On compense ainsi l'augmentation du diamètre du cristallin.

En se référant à nouveau aux figures 4 et 5, on va décrire plus en détail un mode préféré de réalisation du dispositif de traitement 20. La partie centrale de l'anneau 20 référencée 22c est de préférence percée d'orifices 24 angulairement régulièrement espacés et permettant la libre circulation de l'humeur aqueuse de part et d'autres du dispositif dans la chambre antérieure. De même, le bord externe 22a est de préférence constitué par des arcs de cercle tels que 26 séparés par des régions en retrait 28. Les arcs de cercle 26 sont angulairement régulièrement répartis. En conséquence, l'appui sur la paroi interne de l'oeil est réalisé par les seuls secteurs correspondant aux arcs de cercle 26, les portions 28 en retrait permettant également le libre passage de l'humeur aqueuse.

Dans le cas du mode de réalisation de la figure 4, le dispositif de traitement 22 est simplement constitué par une portion d'anneau limitée par des extrémités 30 et 32 laissant une ouverture 34. De préférence, pour faciliter l'insertion de l'anneau dans l'oeil par l'incision réalisée dans la paroi de celui-ci, l'extrémité 30 peut être pourvue d'un prolongement effilé 34.

Dans le cas où le dispositif de traitement 20 est constitué par seulement une portion d'anneau présentant l'ouverture 34, il est possible de réaliser cet anneau en un matériau rigide tel que le PMMA. L'ouverture 34 peut de préférence être comprise entre 30 et 120 degrés assurant ainsi une action suffisante sur les zonules.

Il est également possible d'utiliser un dispositif de traitement constitué par un anneau fermé et donc dépourvu de l'ouverture 34. Dans ce cas bien sûr, il est nécessaire que cet anneau soit réalisé en un matériau souple du type commercialisé sous la marque Hydrogel pour autoriser le pliage de l'anneau autour d'un diamètre en vue de l'introduction du dispositif 20 dans l'oeil à travers une incision de dimension relativement réduite.

Dans le mode de réalisation décrit à titre d'exemple, le diamètre externe D1 est égal à 13,1 mm et le diamètre interne D2 est égal à 9,9 mm. Plus généralement, le diamètre D1 est de préférence compris entre 12,5 et 13,5 mm et le diamètre D2 compris entre 9,5 et 10,5 mm.

Le décalage h entre les deux bords selon la direction de l'axe optique est égal à 1,25 mm afin d'obtenir un déplacement suffisant de la zone médiane des zonules. Plus généralement, ce décalage h est compris entre 0,5 et 2,5 mm et de préférence entre 1 et 1,5 mm.

L'angle d'ouverture 34 entre les deux extrémités de l'anneau est de 35 degrés. Plus généralement, il et compris entre 30 et 120 degrés.

Enfin de préférence, comme le montrent les figures 5 et 5a, les bords respectivement externe et interne de l'anneau 20 ont une forme arrondie. Le rayon de courbure correspondant est de préférence compris entre 0.20 et 0,35 mm afin d'éviter toute lésion des zonules ou de la paroi interne de l'oeil.

## Revendications

1. Dispositif pour traiter la presbytie ou autres affections oculaires liées à un défaut d'accommodation de l'oeil comprenant une pièce (22) ayant sensiblement la forme d'au moins une portion d'anneau et présentant un axe de révolution, **caractérisé en ce que** ladite portion d'anneau a la forme générale d'une portion de tronc de cône et un premier bord (22a) disposé sur un cercle de diamètre D1 compris entre 12,5 et 13,5 mm et un deuxième bord (22b) disposé sur un cercle de diamètre D2 compris entre 9,5 et 10,5 mm, lesdits bords étant décalés selon la direction dudit axe de révolution d'une longueur h comprise entre 0,5 et 2,5 mm, ledit premier bord (22a) étant destiné à être en appui, par au moins une partie de sa longueur, sur une partie de la paroi interne de l'oeil et ledit deuxième bord (22b) étant apte à être appliqué contre une zone médiane (18c) des zonules (18) du cristallin, lesdits premier et deuxième bords étant raccordés entre eux par une partie centrale qui présente une résistance mécanique suffisante pour que le deuxième bord provoque un déplacement de ladite zone médiane desdites zonules provoquant leur mise sous tension et les rendant aptes à provoquer la déformation du cristallin (14) sous l'effet de stimulations appliquées aux dites zonules.

2. Dispositif selon la revendication 1, **caractérisé en ce que** le décalage selon l'axe de révolution, entre le premier bord (22a) et le deuxième bord (22b) est compris entre 1.0 mm et 1,5 mm.

3. Dispositif selon l'une quelconque des revendications 1 et 2, **caractérisé en ce que** ledit premier bord (22a) est constitué par des arcs de cercle (26) séparés par des portions en retrait (28).

4. Dispositif selon la revendication 3, **caractérisé en ce que** lesdits arcs de cercle (26) formant ledit premier bord (22a) sont angulairement régulièrement répartis par rapport audit axe de révolution.

5. Dispositif selon l'une quelconque des revendications 1 à 4, **caractérisé en ce que** ladite pièce (22) a la forme d'un anneau ouvert terminé par deux extrémités (30, 32).

6. Dispositif selon l'une quelconque des revendications 1 à 4, **caractérisé en ce que** ladite pièce (22) a la forme d'un anneau fermé.

7. Dispositif selon l'une quelconque des revendications 1 à 4, **caractérisé en ce que** ladite pièce (22) a la forme d'une portion d'anneau terminée par deux extrémités (30, 32), l'angle au centre entre ces extrémités étant compris entre 30 et 120 degrés.

8. Dispositif selon la revendication 7, **caractérisé en ce que** l'une (30) des deux extrémités (30, 32) de la pièce (22) en forme de portion d'anneau est prolongée par une portion effilée (35) afin de faciliter la mise en place de l'anneau dans l'oeil.

9. Dispositif selon l'une quelconque des revendications 7 et 8, **caractérisé en ce qu'**il est réalisé en un matériau biocompatible rigide.

10. Dispositif selon la revendication 9, **caractérisé en ce qu'**il est réalisé en PMMA.

11. Dispositif selon l'une quelconque des revendications 1 à 8, **caractérisé en ce qu'**il est réalisé en un matériau biocompatible souple.

12. Dispositif selon l'une quelconque des revendications 1 à 11, **caractérisé en ce que** les bords (22a, 22b) dudit anneau sont arrondis.

## Patentansprüche

1. Vorrichtung zur Behandlung der Weitsichtigkeit oder anderer Augenbeschwerden, die mit einem Akkommodationsfehler des Auges verbunden sind, die ein im Wesentlichen die Form mindestens eines Ringteils besitzendes und eine Umdrehungsachse aufweisendes Teil (22) umfasst, **dadurch gekennzeichnet, dass** das Ringteil die allgemeine Form eines Kegelstumpfteils hat und einen auf einem Kreis mit Durchmesser D1 zwischen 12,5 und 13,5 mm angeordneten ersten Rand (22a) und einen auf einem Kreis mit Durchmesser D2 zwischen 9,5 und 10,5 mm angeordneten zweiten Rand (22b) besitzt, wobei die Ränder in eine Richtung der Umdrehungsachse um eine Länge h zwischen 0,5 und 2,5 mm versetzt sind, wobei der erste Rand (22a) dazu bestimmt ist, mit mindestens einem Teil seiner Länge auf einem Teil der inneren Wand des Auges aufzuliegen, und der zweite Rand (22b) gegen eine mittlere Zone (18c) der Zonulae (18) der Augenlinse angelegt werden kann, wobei der erste und der zweite Rand untereinander über ein zentrales Teil verbunden sind, das einen mechanischen Widerstand aufweist, der ausreicht, dass der zweite Rand ein Verschieben der mittleren Zone der Zonulae bewirkt, was ihr Anspannen verursacht und sie geeignet macht, die Verformung der Augenlinse (14) unter der Einwirkung von Stimulationen zu bewirken, die auf die Zonulae angewandt werden.

2. Vorrichtung nach Anspruch 1, **dadurch gekennzeichnet, dass** das Verschieben entlang der Umdrehungsachse zwischen dem ersten Rand (22a) und dem zweiten Rand (22b) zwischen 1,0 mm und 1,5 mm liegt.

3. Vorrichtung nach einem der Ansprüche 1 und 2, **dadurch gekennzeichnet, dass** der erste Rand (22a) aus Kreisbögen (26) besteht, die durch rückspringende Teile (28) getrennt werden.

4. Vorrichtung nach Anspruch 3, **dadurch gekennzeichnet, dass** die Kreisbögen (26), die den ersten Rand (22a) bilden, winkelig regelmäßig zu der Umdrehungsachse verteilt sind.

5. Vorrichtung nach einem der Ansprüche 1 bis 4, **dadurch gekennzeichnet, dass** das Teil (22) die Form eines offenen Rings hat, der von zwei Enden (30, 32) abgeschlossen wird.

6. Vorrichtung nach einem der Ansprüche 1 bis 4, **dadurch gekennzeichnet, dass** das Teil (22) die Form eines geschlossenen Rings hat.

7. Vorrichtung nach einem der Ansprüche 1 bis 4, **dadurch gekennzeichnet, dass** das Teil (22) die Form eines Ringteils hat, das von zwei Enden (30, 32) abgeschlossen wird, wobei der Winkel in der Mitte zwischen diesen Enden zwischen 30 und 120 Grad liegt.

8. Vorrichtung nach Anspruch 7, **dadurch gekennzeichnet, dass** eines (30) der zwei Enden (30, 32) des Teils (22) in Ringteilform von einem zugespitzten Teil (35) verlängert wird, um das Anbringen des Rings im Auge zu erleichtern.

9. Vorrichtung nach einem der Ansprüche 7 und 8, **dadurch gekennzeichnet, dass** sie aus einem starren, biokompatiblen Werkstoff hergestellt ist.

10. Vorrichtung nach Anspruch 9, **dadurch gekennzeichnet, dass** sie aus PMMA besteht.

11. Vorrichtung nach einem der Ansprüche 1 bis 8, **dadurch gekennzeichnet, dass** sie aus einem geschmeidigen biokompatiblen Werkstoff hergestellt ist.

12. Vorrichtung nach einem der Ansprüche 1 bis 11, **dadurch gekennzeichnet, dass** die Ränder (22a, 22b) des Rings abgerundet sind.

## Claims

1. Device for treating presbyopia or other ocular disorder related to an eye accommodation defect comprising a piece (22) having substantially the shape of at least a ring portion with an axis of revolution, the device being **characterised in that** said ring portion has the general shape of a portion of a truncated cone, and a first edge (22a) disposed on a circle of diameter D1 included between 12.5 and 13.5 mm and a second edge (22b) disposed on a circle of diameter D2 included between 9.5 and 10.5 mm, said edges being offset in the direction of said axis of revolution by a length h included between 0.5 and 2.5 mm, said first edge (22a) being designed to rest, over at least part of its length, on part of the internal wall of the eye and said second edge (22b) being capable of being pressed against a median zone (18c) of the zonules (18) of the crystalline lens, said first and second edges being joined together by a central part which presents a sufficient mechanical strength for the second edge to provoke a displacement of said median zone of said zonules stressing them and enabling them to cause the deformation of the crystalline lens (14) under the effect of stimulations applied to said zonules.

2. Device according to Claim 1, **characterised in that** the offset in the direction of the axis of revolution, between the first edge (22a) and the second edge (22b), is included between 1.0 mm and 1.5 mm.

3. Device according to Claim 1 or 2, **characterised in that** said first edge (22a) is constituted by arcs of circle (26) separated by recessed portions (28).

4. Device according to Claim 3, **characterised in that** said arcs of circle (26) forming said first edge (22a) are regularly distributed angularly with respect to said axis of revolution.

5. Device according to any one of Claims 1 to 4, **characterised in that** said piece (22) is in the form of an open ring terminating in two ends (30, 32).

6. Device according to any one of Claims 1 to 4, **characterised in that** said piece (22) is in the form of a closed ring.

7. Device according to any one of Claims 1 to 4, **characterised in that** said piece (22) is in the form of a ring portion terminating in two ends (30, 32), the angle at the centre between these ends being included between 30 and 120 degrees.

8. Device according to Claim 7, **characterised in that** one (30) of the two ends (30, 32) of the piece (22) in the form of ring portion is extended by an elongated portion (35) in order to facilitate positioning of the ring in the eye.

9. Device according to Claim 7 or 8, **characterised in that** it is made of a rigid biocompatible material.

10. Device according to Claim 9, **characterised in that** it is made of PMMA.

11. Device according to any one of Claims 1 to 8, **characterised in that** it is made of a supple biocompatible material.

12. Device according to any one of Claims 1 to 11, **characterised in that** the edges (22a, 22b) of said ring are rounded.
